# EUROPEAN PATENT APPLICATION

(11) **EP 4 810 018 A1**
(43) Date of publication of application: **23.09.2026**
(21) Application number: 25211134.9
(22) Date of filing: 24.10.2025
(51) Int. Cl.: A61K 9/00, A61K 9/08, A61K 31/165, A61K 47/12, A61K 47/14, A61K 47/26

(54) **AN ORAL SOLUTION FORMULATION OF LISDEXAMFETAMINE DIMESYLATE**

(30) Priority: 20.03.2025 TR 2025003340
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); YUCEL, Alpay, Istanbul (TR)
(74) Representative: Genç Ilhan, Oznur

(57) **Abstract**

The present invention relates to an oral solution formulation comprising lisdexamfetamine dimesylate and at least one antimicrobial preservative and at least one buffering agent wherein a weight ratio of lisdexamfetamine dimesylate to buffering agent is between 1 and 7. Furthermore, the oral solution formulation is also obtained by using an effective process which is simple, rapid, cost effective, time-saving and industrially convenient process.

## Description

### Field of the Invention

The present invention relates to an oral solution formulation comprising lisdexamfetamine dimesylate and at least one antimicrobial preservative and at least one buffering agent wherein a weight ratio of lisdexamfetamine dimesylate to buffering agent is between 1 and 7. Furthermore, the oral solution formulation is also obtained by using an effective process which is simple, rapid, cost effective, time-saving and industrially convenient process.

### Background of the Invention

Lisdexamfetamine dimesylate is used to treat attention deficit hyperactivity disorder (ADHD) in adults and children 6 years of age and older. This medicine is also used to treat moderate to severe binge eating disorder (BED). It belongs to the group of medicines called central nervous system (CNS) stimulants.

Lisdexamfetamine dimesylate increases attention and decreases restlessness in children and adults who are overactive, cannot concentrate for very long, or are easily distracted and impulsive. This medicine is used as part of a total treatment program that also includes social, educational, and psychological treatment.

Lisdexamfetamine dimesylate is also referred to as I-lysine-d-amphetamine dimesylate of Formula I is chemically named as (2S)-2,6-Diamino-N-[(1S)-1-methyl-2-phenylethyl]-hexanamide dimesylate.

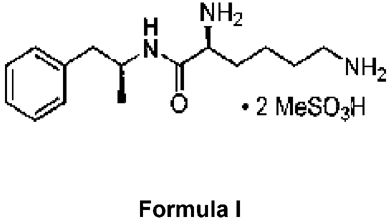

Lisdexamfetamine dimesylate is a white to off-white powder, very soluble in water and soluble in methanol, melting point 192-198°C, molecular formula C₁₇H₃₃N₃O₇S₂ and molecular weight 455.59 g/mol.

Lisdexamfetamine is currently commercially available only as 10, 20, 30, 40, 50, 60 or 70 mg hard capsules or chewable tablets. Each capsule or chewable tablet contains 10, 20, 30, 40, 50, 60 or 70 mg lisdexamfetamine dimesylate, equivalent to 5.8 mg, 11.6 mg, 17.3 mg, 23.1 mg, 28.9 mg, 34.7 mg or 40.5 mg lisdexamfetamine, respectively.

The patent WO2021136602 discloses an oral pharmaceutical solution comprising a pharmaceutically acceptable lisdexamfetamine salt and a pharmaceutically acceptable aqueous carrier comprising a buffer and a cosolvent selected from the group consisting of a glycol, a polyol and a mixture thereof, wherein the pH of the solution is between 5.5 and 9.0.

The patent EP4279062 discloses new liquid compositions of lisdexamfetamine, their uses, kits and treatment methods for treating attention deficit hyperactivity disorder (ADHD) and binge eating disorder (BED).

Oral solution formulations of lisdexamfetamine dimesylate are known to have disadvantages compared to capsules and tablets. Lisdexamfetamine dimesylate may be sensitive to heat or light, which may shorten its shelf life. Oral solution formulations are more unstable and prone to degradation than capsules and tablets. However, in terms of patient compliance, there is a need for a formulation that is highly stable, easy to use and easily adjusted to the required dose, and supports compliance with treatment in patients having swallowing difficulties and children.

We overcame all these disadvantages by using an antimicrobial preservative and a buffering agent in our oral solution formulation and also by obtaining a formulation with surprisingly high stability when the weight ratio of lisdexamfetamine dimesylate to the buffering agent was between 1 and 7.

### Detailed Description of the Invention

The main object of the present invention is to provide an oral solution formulation comprising lisdexamfetamine dimesylate with high stability.

Another object of the present invention is to provide an oral solution formulation comprising lisdexamfetamine dimesylate having homogeneity.

Another object of the present invention is to provide a method for an oral solution formulation comprising lisdexamfetamine dimesylate prepared by simple, easy, time-saving and fast manufacturing methods.

Lisdexamfetamine dimesylate oral solutions are known to be more unstable. Therefore, the use of antimicrobial preservatives and buffering agents in lisdexamfetamine dimesylate oral solution formulations may help to increase stability and maintain the efficacy of lisdexamfetamine dimesylate in particular. The combined use of these two excipients may minimize some of the disadvantages of lisdexamfetamine dimesylate (e.g. which may shorten its shelf life or stability problems due to pH changes). In the oral solution formulation of lisdexamfetamine dimesylate, it is very important that the drug is evenly distributed. It is important to create a formulation considering all these disadvantages of lisdexamfetamine dimesylate oral solution formulations. In this formulation, we used a combination of antimicrobial preservative and buffer agent and the weight ratio of lisdexamfetamine dimesylate to buffer agent was between 1 and 7. Surprisingly, it was seen that both high stability and homogeneous distribution of the active ingredient were achieved.

According to one embodiment of the invention, an oral solution formulation comprising lisdexamfetamine dimesylate and at least one antimicrobial preservative and at least one buffering agent wherein a weight ratio of lisdexamfetamine dimesylate to buffering agent is between 1 and 7.

According to one embodiment of the present invention, the amount of the lisdexamfetamine dimesylate is between 0.1% and 10.0% by weight of the total formulation. Preferably, it is between 0.5% and 7.0% by weight of the total formulation.

Suitable antimicrobial preservatives are selected from the group comprising sodium benzoate, methylparaben, alpha tocopherol (Vitamin E), quercetin, butyl hydroxyanisole (BHA), butylhydroxytoluene (BHT), erythorbic acid, monothioglycerol, potassium metabisulfite, alpha lipoic acid, ascorbyl palmitate, propyl gallate, sodium ascorbate, sodium metabisulfite, sodium sulfite, thymol or mixtures thereof.

According to this embodiment of the present invention, the antimicrobial preservative is sodium benzoate or methylparaben or mixture thereof.

According to one embodiment of the present invention, the amount of the antimicrobial preservative is between 0.001% and 5.0% by weight in the total formulation.

Suitable buffering agents are selected from the group comprising sodium dihydrogen phosphate, disodium hydrogen phosphate, citric acid anhydrous, trisodium citrate dihydrate, sodium carbonate, alkali metal citrate, sodium citrate, tartaric acid, fumaric acid, sorbic acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate or mixtures thereof.

According to this embodiment of the present invention, the buffering agent is sodium dihydrogen phosphate or disodium hydrogen phosphate or citric acid anhydrous or trisodium citrate dihydrate or mixture thereof.

According to this embodiment of the present invention, the buffering agent is sodium dihydrogen phosphate and disodium hydrogen phosphate.

According to this embodiment of the present invention, the buffering agent is citric acid anhydrous and trisodium citrate dihydrate.

According to one embodiment of the present invention, the amount of the buffering agent is between 0.01% and 3.0% by weight in the total formulation.

According to one embodiment of the present invention, the oral solution formulation further comprises at least one pharmaceutically acceptable excipient selected from the group comprising sweeteners, viscosity-increasing agents, flavouring agents, solvents or mixtures thereof.

Suitable sweeteners are selected from the group comprising xylitol, sucralose, sucrose, potassium acesulfame, sodium saccharinate, neohesperidine dihydrochalcone, monoammonium glycyrrhizinate, saccharin, glucose, lactose, fructose, mannitol, erythritol or mixtures thereof.

According to this embodiment of the present invention, the sweetener is xylitol or sucralose or mixture thereof.

According to one embodiment of the present invention, the amount of the sweetener is between 0.1% and 15.0% by weight in the total formulation.

Suitable viscosity-increasing agents are selected from the group comprising carboxymethylcellulose sodium, hydrophobic colloidal silica, hydroxyethyl cellulose, hydroxyethylmethyl cellulose, hydroxypropyl cellulose, hypromellose, magnesium aluminum silicate, methylcellulose, polyvinyl alcohol, glyceryl behenate, gelatin or mixture thereof.

According to this embodiment of the present invention, the viscosity-increasing agent is carboxymethylcellulose sodium.

According to one embodiment of the present invention, the amount of the viscosity-increasing agent is between 0.01% and 5.0% by weight in the total formulation.

Suitable flavouring agents are selected from the group comprising raspberry, lemon cream, peppermint, menthol, orange, cherry, cinnamon, chocolate, vanillin, strawberry, grape, black currant, banana, red fruits, wild berries or mixtures thereof.

According to this embodiment of the present invention, the flavouring agent is raspberry.

According to one embodiment of the present invention, the amount of the flavouring agent is between 0.01% and 5.0% by weight in the total formulation.

Suitable solvents are selected from the group comprising purified water, sorbitol, glycerin, propylene glycol, ethanol, polyethylene glycol or mixtures thereof.

According to this embodiment of the invention, a process for an oral solution formulation comprising lisdexamfetamine dimesylate comprises the following process steps;
a. Mixing purified water and buffering agents until a homogeneous solution is obtained,
b. Adding antimicrobial preservative to the mixture in step (a) and mixing the mixture until a homogeneous solution is obtained,
c. Adding lisdexamfetamine dimesylate, sweetener, flavouring agent and solvent to the mixture of step (b) and mixing the mixture until a homogeneous solution is obtained,
d. Adjusting the pH of the solution to the desired value,
e. Completing of the solution to volume with purified water.

### Example 1;

| **Ingredients** | **%by weight** | **%by weight** |
|---|---|---|
| Lisdexamfetamine Dimesylate | 4 | 0.5-10 |
| Sodium benzoate | 0.01 | 0.001-5 |
| Sodium dihydrogen phosphate | 0.4 | 0.01-5 |
| Disodium hydrogen phosphate | 0.2 | 0.01-5 |
| Xylitol | 5 | 0.5-10 |
| Sucralose | 1.5 | 0.1-10 |
| Sorbitol | 35 | 25-45 |
| Raspberry flavour | 0.5 | 0.01-5 |
| Glycerin | 25 | 15-35 |
| Water q.s.p | q.s | q.s |
| **TOTAL** | **100** | **100** |

| | | |
|---|---|---|
| q.s: quantity sufficient | | |

A process for example 1;
a. Mixing 90% purified water, sodium dihydrogen phosphate and disodium hydrogen phosphate until a homogeneous solution is obtained,
b. Adding sodium benzoate to the mixture in step (a) and mixing the mixture until a homogeneous solution is obtained,
c. Adding lisdexamfetamine dimesylate, xylitol, sucralose, sorbitol, raspberry flavour and glycerin to the mixture of step (b) and mixing the mixture until a homogeneous solution is obtained,
d. Adjusting the pH of the solution to the desired value,
e. Completing the solution to volume with the remaining 10% amount of purified water.

### Example 2;

| **Ingredients** | **%by weight** | **%by weight** |
|---|---|---|
| Lisdexamfetamine Dimesylate | 1 | 0.1-10 |
| Trisodium citrate dihydrate | 0.1 | 0.01-5 |
| Citric acid anhydrous | 0.5 | 0.01-5 |
| Methyl Paraben | 0.1 | 0.01-5 |
| Carboxymethylcellulose Sodium | 0.2 | 0.01-5 |
| Sucralose | 3 | 0.1-10 |
| Sorbitol | 22 | 10-30 |
| Glycerin | 15 | 5-25 |
| Raspberry flavour | 0.3 | 0.01-5 |
| Water q.s.p | q.s | q.s |
| **TOTAL** | **100** | **100** |

| | | |
|---|---|---|
| q.s: quantity sufficient | | |

A process for example 2;
a. Mixing 90% purified water, trisodium citrate dihydrate and citric acid anhydrous phosphate until a homogeneous solution is obtained,
b. Heating the solution to 80°C,
c. Adding methyl paraben to the mixture in step (b) and mixing the mixture until a homogeneous solution is obtained,
d. After dissolving methyl paraben, cool the solution to room temperature,
e. Adding lisdexamfetamine dimesylate, sucralose, sorbitol, carboxymethylcellulose sodium, raspberry flavour and glycerin to the mixture of step (d) and mixing the mixture until a homogeneous solution is obtained,
f. Adjusting the pH of the solution to the desired value,
g. Completing the solution to volume with the remaining 10% amount of purified water.

## Claims

1. An oral solution formulation comprising lisdexamfetamine dimesylate and at least one antimicrobial preservative and at least one buffering agent, wherein a weight ratio of lisdexamfetamine dimesylate to buffering agent is between 1 and 7.

2. The oral solution formulation according to claim 1, wherein the amount of the lisdexamfetamine dimesylate is between 0.1% and 10.0% by weight of the total formulation.

3. The oral solution formulation according to claim 1, wherein antimicrobial preservatives are selected from the group comprising sodium benzoate, methylparaben, alpha tocopherol (Vitamin E), quercetin, butyl hydroxyanisole (BHA), butylhydroxytoluene (BHT), erythorbic acid, monothioglycerol, potassium metabisulfite, alpha lipoic acid, ascorbyl palmitate, propyl gallate, sodium ascorbate, sodium metabisulfite, sodium sulfite, thymol or mixtures thereof.

4. The oral solution formulation according to claim 1 or 3, wherein the antimicrobial preservative is sodium benzoate or methylparaben or mixture thereof.

5. The oral solution formulation according to claim 3 or 4, wherein the amount of the antimicrobial preservative is between 0.001% and 5.0% by weight in the total formulation.

6. The oral solution formulation according to claim 1, wherein buffering agents are selected from the group comprising sodium dihydrogen phosphate, disodium hydrogen phosphate, citric acid anhydrous, trisodium citrate dihydrate, sodium carbonate, alkali metal citrate, sodium citrate, tartaric acid, fumaric acid, sorbic acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate or mixtures thereof.

7. The oral solution formulation according to claim 1 or 6, wherein the buffering agent is sodium dihydrogen phosphate or disodium hydrogen phosphate or citric acid anhydrous or trisodium citrate dihydrate or mixture thereof.

8. The oral solution formulation according to claim 6 or 7, wherein the amount of the buffering agent is between 0.01% and 3.0% by weight in the total formulation.

9. The oral solution formulation according to claim 1, wherein the oral solution formulation further comprises at least one pharmaceutically acceptable excipient selected from the group comprising sweeteners, viscosity-increasing agents, flavouring agents, solvents or mixtures thereof.

10. The oral solution formulation according to claim 9, wherein sweeteners are selected from the group comprising xylitol, sucralose, sucrose, potassium acesulfame, sodium saccharinate, neohesperidine dihydrochalcone, monoammonium glycyrrhizinate, saccharin, glucose, lactose, fructose, mannitol, erythritol or mixtures thereof.

11. The oral solution formulation according to claim 10, wherein the sweetener is xylitol or sucralose or mixture thereof.

12. The oral solution formulation according to claim 9, wherein viscosity-increasing agents are selected from the group comprising carboxymethylcellulose sodium, hydrophobic colloidal silica, hydroxyethyl cellulose, hydroxyethylmethyl cellulose, hydroxypropyl cellulose, hypromellose, magnesium aluminum silicate, methylcellulose, polyvinyl alcohol, glyceryl behenate, gelatin or mixture thereof.

13. The oral solution formulation according to claim 12, wherein the viscosity-increasing agent is carboxymethylcellulose sodium.

14. The oral solution formulation according to claim 9, wherein flavouring agents are selected from the group comprising raspberry, lemon cream, peppermint, menthol, orange, cherry, cinnamon, chocolate, vanillin, strawberry, grape, black currant, banana, red fruits, wild berries or mixtures thereof.

15. A process for an oral solution formulation comprising lisdexamfetamine dimesylate according to any of the precedings claims comprising the following process steps;
a. Mixing purified water and buffering agents until a homogeneous solution is obtained,
b. Adding antimicrobial preservative to the mixture in step (a) and mixing the mixture until a homogeneous solution is obtained,
c. Adding lisdexamfetamine dimesylate, sweetener, flavouring agent and solvent to the mixture of step (b) and mixing the mixture until a homogeneous solution is obtained,
d. Adjusting the pH of the solution to the desired value,
e. Completing of the solution to volume with purified water.
